# FASCICULE DE BREVET EUROPEEN

(11) **EP 3 531 986 B1**
(45) Date de publication et mention de la délivrance du brevet: **13.01.2021**
(21) Numéro de dépôt: 17801076.5
(22) Date de dépôt: 27.10.2017
(51) Int. Cl.: A61F 2/954, A61F 2/97

(54) **LANCEURS ET ENSEMBLE POUR LA POSE D'IMPLANTS CONNECTABLES**
TREIBER UND SET ZUM EINSETZEN VON VERBINDBAREN IMPLANTATEN
DRIVERS AND SET FOR THE INSERTION OF CONNECTABLE IMPLANTS

(30) Priorité: 27.10.2016 FR 1660463
(43) Date de publication de la demande: 04.09.2019
(73) Titulaire: BS Medical Tech Industry, 67470 Niederroedern (FR); ID Nest Medical, 67000 Strasbourg (FR)
(72) Inventeur: BASCH, Bertrand, 67620 Soufflenheim (FR); CONTASSOT, David, 67100 Strasbourg (FR)
(74) Mandataire: Merckling, Norbert
(86) Numéro de dépôt international: PCT/FR2017/052969
(87) Numéro de publication internationale: WO 2018/078297

(56) Documents cités:
- EP-A1- 3 037 072
- WO-A1-99/34749
- WO-A1-2007/124053
- US-A1- 2012 271 410

## Description

### Domaine technique

La présente invention se rapporte au domaine technique général de la mise en place d'implants connectables dans tous conduits de circulation d'un fluide corporel comportant une ou plusieurs bifurcations. Par connectable, il convient d'entendre des implants pouvant être liés mécaniquement, une fois posés dans les conduits de la bifurcation.

L'invention concerne plus particulièrement des moyens destinés par exemple à la mise en place d'implants, du genre stents connectables, au niveau de la confluence des veines iliaques, ou encore d'endoprothèses connectées au niveau de la crosse aortique. Ces moyens sont appelés « lanceurs », mais également « dispositifs ou systèmes de pose ». Quant aux implants, il peut s'agir par exemple de stents auto-expansibles ou expansibles sur ballonet ou de manière générale tout type de stents existants.

L'invention trouve également son application en urologie.

Ces moyens sont destinés par exemple à mettre en place des implants connectés dans une cavité d'un être vivant et tout particulièrement dans le réseau circulatoire et notamment artériel et veineux, dans une région où le vaisseau correspondant présente des branches collatérales nécessitant le maintien de leur perfusion.

Dans ce cas un premier dispositif permet la mise en place de l'implant tubulaire mère dans un conduit de circulation du sang tel que l'aorte.

Un second dispositif permet le déploiement de branches de quelque forme et caractéristique que ce soit, pour permettre par exemple la vascularisation de l'artère iliaque interne au niveau de l'aorte thoraco-abdominale et de l'artère carotide primitive gauche, de l'artère sous-clavière gauche et du tronc artériel brachio-céphalique au niveau de la crosse aortique et enfin des artères coronaires au niveau de la racine de l'aorte.

De même, un tel dispositif s'applique à toutes les bifurcations de type veineuses telles que, la veine cave inférieure et ses branches iliaques et rénales, ainsi que la veine cave supérieure et ses branches.

D'autres applications sont aussi possibles dans tout autre conduit de circulation d'un fluide corporel comportant une ou plusieurs bifurcations.

De tels moyens sont généralement utilisés pour la mise en place d'implants en cas de maladie vasculaire tel que les anévrismes, les dissections, les lésions oblitérantes et les syndromes compressifs.

Les implantations se font généralement à l'aide de techniques endoluminales. Ces techniques mini-invasives, procurent moins de mortalité et morbidité pour le patient, ainsi qu'une diminution du temps opératoire.

### Etat de la technique

On connaît par exemple par le document EP 1 648 343 un système pour traiter une bifurcation vasculaire. Le système décrit comporte un cathéter à deux branches permettant d'implanter un stent dans chacun des conduits de la bifurcation. Un tel système présente l'inconvénient d'être constitué d'un nombre important d'éléments constitutifs lesquels devront être introduits dans l'un des conduits. L'assemblage d'un tel système est donc complexe sur le plan de la fabrication. En outre, cela a une incidence non négligeable sur son encombrement, ce qui n'est pas souhaitable. L'utilisation d'un tel système complexe rend également l'intervention chirurgicale plus complexe, pouvant aboutir à des mauvaises manipulations ou à un positionnement des implants moins fiable. La mise en place de guides n'est pas ailleurs pas faite en première intention et reste complexe et aléatoire du fait de l'encombrement du conduit par le stent principal déjà implanté.

On connait également, par l'intermédiaire du document US 2012/0271410 A1, un système d'implantation de deux stents au niveau d'une bifurcation. Le système, inclut un élément de couverture réalisant la liaison mécanique et un pré-assemblage des stents avant l'implantation. Ce système n'est donc pas adapté pour connecter des implants non pourvus d'éléments de couverture. En outre, ce système ne comprend qu'un unique lanceur délivrant un stent principal et un stent branche et les éléments de couverture. L'encombrement dimensionnel nécessaire d'un tel lanceur est donc défavorablement augmenté. L'utilisation d'un lanceur unique, lequel permet aussi de positionner des guides dans les conduits, altère la maniabilité des divers éléments, notamment les stents et les gaines lors de l'opération de pose. En effet, tout déplacement d'un stent induit obligatoirement un déplacement sur l'autre stent. L'opération de pose de stents est ainsi rendue plus compliquée, moins précise et par conséquent moins fiable.

### Exposé de l'invention

Le but de la présente invention vise à pallier les inconvénients de l'état de la technique et à proposer un nouvel ensemble de pose permettant de réaliser in situ la connexion entre un implant tubulaire primaire et au moins un implant tubulaire secondaire de façon simple, précise et fiable.

Un nouveau procédé de positionnement et de connexion d'implants, dont la mise en œuvre est simple et fiable, est proposé.

Les objets assignés à l'invention sont atteints à l'aide d'un lanceur principal comportant au moins un implant principal pour la mise en place dudit au moins un implant connectable à un autre implant, au niveau d'une bifurcation entre un conduit principal et un conduit secondaire de circulation principal d'un fluide corporel, ledit lanceur principal présentant une extrémité distale D et une extrémité proximale P de manipulation comportant un point fixe,
caractérisé en ce que:
- ledit lanceur principal comporte une gaine intérieure pourvue à son extrémité distale D d'une pointe a-traumatique avec un trou axial communiquant avec un tube longitudinal formé par ladite gaine intérieure,
- ledit lanceur principal comportant une gaine extérieure,
- ledit au moins un implant principal étant disposé entre les gaines extérieure et intérieure,
- la gaine extérieure principale étant pourvue d'une rainure ou fente longitudinale s'étendant sur une partie de la longueur,
- la gaine intérieure principale présentant un trou latéral débouchant en regard de ladite rainure ou fente longitudinale,
- ledit implant principal présentant au moins une fenêtre alignée avec le trou latéral,
- et ledit lanceur principal comportant au moins une gaine complémentaire dont une première extrémité traverse le trou latéral, la fenêtre de l'implant principal et la rainure ou fente longitudinale et dont une seconde extrémité sort au voisinage de l'extrémité proximale P, ladite gaine complémentaire étant destinée à être traversée par un guide.

Selon un exemple de réalisation du lanceur conforme à l'invention, la gaine extérieure principale est montée coulissante sur la gaine intérieure principale et bloquée en coulissement par la pointe a-traumatique d'une part et par au moins une butée amovible d'autre part, ladite rainure ou fente longitudinale étant ouverte à son extrémité distale au voisinage de la pointe a-traumatique.

Selon un exemple de réalisation du lanceur conforme à l'invention, la gaine extérieure principale est constituée d'un matériau pelable.

Selon un exemple de réalisation du lanceur conforme à l'invention, la gaine complémentaire est extractible du lanceur principal et permet l'introduction d'un guide secondaire dans ledit lanceur principal et permet d'assurer le passage dans la fenêtre de l'implant principal et le passage du trou latéral de la gaine intérieure.

Les objets assignés à l'invention sont atteints également à l'aide d'un ensemble pour la mise en place et la connexion d'au moins deux implants dans des conduits de circulation principal et secondaire d'un fluide corporel, le conduit principal présentant avec le conduit secondaire une bifurcation, caractérisé en ce qu'il comprend :
- un lanceur principal tel que présenté ci-dessus,
- un lanceur secondaire, indépendant du lanceur principal, présentant une extrémité distale D et une extrémité proximale P de manipulation comportant un point fixe,
- ledit lanceur secondaire comportant une gaine secondaire intérieure pourvue à son extrémité distale D, d'une pointe a-traumatique secondaire avec un trou axial secondaire communiquant avec un tube longitudinal formé par ladite gaine secondaire intérieure, une gaine secondaire extérieure, au moins un implant secondaire disposé entre les gaines secondaires extérieure et intérieure.

Selon un exemple de réalisation, l'ensemble conforme à l'invention comprend un guide principal pré-implantable dans le conduit principal et destiné à s'engager dans le trou axial à des fins de guidage du lanceur principal.

Selon un exemple de réalisation, l'ensemble conforme à l'invention comprend un guide secondaire pré-implantable dans le conduit principal et dans le conduit secondaire en traversant la bifurcation, pour s'engager dans la gaine complémentaire à des fins de positionnement du lanceur principal et/ou à des fins de guidage du lanceur secondaire.

Selon un exemple de réalisation de l'ensemble conforme à l'invention, la pointe a-traumatique secondaire du lanceur secondaire est une pointe à double cône.

Selon un exemple de réalisation de l'ensemble conforme à l'invention, la gaine extérieure secondaire est montée coulissante sur la gaine intérieure secondaire et bloquée en coulissement par la pointe a-traumatique secondaire d'une part et par au moins deux butées successives et amovibles A, B d'autre part, lesdites butées A, B assurant la libération progressive de l'implant secondaire ainsi que sa connexion sur l'implant principal.

Selon un autre exemple de réalisation de l'ensemble conforme à l'invention, la gaine extérieure secondaire est montée coulissante sur la gaine intérieure secondaire et bloquée en coulissement par la pointe a-traumatique secondaire d'une part et par trois butées successives et amovibles A, B, C d'autre part.

Selon un exemple de réalisation de l'ensemble conforme à l'invention, l'implant secondaire est conformé pour présenter des organes de connexion à l'implant principal, lesquels forment la connexion avec l'implant principal automatiquement lors de la libération et du déploiement dudit implant secondaire.

Selon un exemple de réalisation de l'ensemble conforme à l'invention la pointe a-traumatique secondaire présente une extrémité distale D incurvée.

Selon un exemple de réalisation de l'ensemble conforme à l'invention, la pointe a-traumatique secondaire présente est réalisée avec un matériau flexible.

Selon un exemple de réalisation de l'ensemble conforme à l'invention, les implants sont reliés à la gaine intérieure correspondante par l'intermédiaire d'un organe de retenue permettant de retirer et/ou de repositionner dans le lanceur lesdits implants en cas de nécessité lors de l'opération de pose.

Les objets assignés à l'invention sont également atteints à l'aide d'un procédé de mise en place et de connexion in situ de deux implants dans des conduits de circulation principal et secondaire d'un fluide corporel, le conduit de circulation principal présentant avec le conduit de circulation secondaire une bifurcation, ledit procédé étant mis en œuvre avec un ensemble selon la présentation ci-dessous, caractérisé en ce qu'il comprend les étapes :
- engager un guide secondaire dans le conduit secondaire,
- introduire dans le conduit principal, un organe de préhension et accrocher le guide secondaire au niveau de la bifurcation,
- faire passer l'extrémité du guide secondaire dans le conduit principal et extraire une extrémité de celui-ci du conduit principal,
- engager un guide principal dans le conduit principal,
- engager l'extrémité du guide secondaire, sortant du conduit principal, dans la première extrémité de la gaine complémentaire du lanceur principal,
- engager l'extrémité du guide principal sortant du conduit principal dans le trou axial du lanceur principal,
- retirer la gaine complémentaire du lanceur principal,
- introduire le lanceur principal et le positionner au niveau de la bifurcation à l'aide du guide principal,
- faire coulisser la gaine extérieure sur la gaine intérieure pour libérer l'implant principal et déployer l'implant principal,
- introduire le lanceur secondaire dans le conduit secondaire jusqu'au niveau de l'implant principal à l'aide du guide secondaire,
- faire coulisser la gaine secondaire extérieure sur la gaine secondaire intérieure pour libérer progressivement l'implant secondaire, le déployer et le connecter à l'implant principal,
- retirer des conduits les lanceurs principal et secondaire ainsi que les guides principal et secondaire,

Selon un exemple de mise en œuvre conforme à l'invention, le procédé consiste à faire coulisser la gaine secondaire extérieure et à utiliser un système de butées amovibles pour bloquer en coulissement la gaine secondaire extérieure dans des positions successives correspondant à la libération progressive de l'implant secondaire ainsi qu'à sa connexion à l'implant principal.

Selon un exemple de mise en œuvre conforme à l'invention, le procédé consiste à repositionner l'implant secondaire (13) dans le lanceur secondaire (2) en vue d'une libération et d'une mise en place à un autre emplacement plus adapté dans le conduit secondaire (C2).

L'ensemble conforme à l'invention présente l'avantage d'être simple à utiliser et à manipuler tout en garantissant une fiabilité remarquable dans le positionnement des implants. En effet, l'utilisation de deux lanceurs distincts, introduits dans les conduits respectifs via des incisions distinctes, permet de manipuler individuellement chaque implant. Cela augmente la précision du largage des implants et la fiabilité de la connexion entre les implants.

Un autre avantage de l'ensemble conforme à l'invention réside dans l'utilisation de chacun des conduits de la bifurcation pour l'insertion d'un lanceur. Chacun des lanceurs, ne comportant qu'un nombre réduit d'éléments peut donc présenter un encombrement faible et simplifier les gestes chirurgicaux.

Un autre avantage de l'ensemble conforme à l'invention et plus particulièrement du procédé de pose et de connexion conforme à l'invention, réside dans l'obtention d'une précision et d'une fiabilité remarquables des gestes opératoires.

Un autre avantage du lanceur ou de l'ensemble conforme à l'invention, réside dans l'absence d'arêtes saillantes susceptibles d'accrocher les implants après leur déploiement.

Les coûts de fabrication de l'ensemble conforme à l'invention sont par ailleurs réduits par rapport aux coûts des systèmes connus. En outre, avec un ensemble conforme à l'invention, le site d'implantation est parfaitement maîtrisé, la durée d'intervention est réduite et les risques opératoires sont réduits.

### Brève description des figures

D'autres avantages et particularités de l'invention ressortiront également à la lecture des dessins annexés, lesquels sont donnés à titre d'exemples illustratifs et non limitatifs dans lesquels :
- la figure 1, est une illustration d'un exemple de réalisation d'un lanceur principal conforme à l'invention,
- la figure 2, est un agrandissement partiel du lanceur principal de la figure 1,
- la figure 3, est une représentation d'un détail agrandi des figures 1 ou 2,
- la figure 4, est une représentation du lanceur principal de la figure 1, prêt à être utilisé pour une intervention chirurgicale,
- les figures 5 et 6, illustrent deux autres modes de réalisation du lanceur principal conforme à l'invention,
- la figure 7, est une représentation d'un exemple de réalisation d'un lanceur secondaire d'un ensemble conforme à l'invention,
- la figure 8, est un agrandissement partiel du lanceur secondaire de la figure 7,
- la figure 9, est une représentation du lanceur secondaire de la figure 7, prêt à être utilisé pour une intervention chirurgicale,
- la figure 10 est une illustration schématique agrandie de l'extrémité distale du lanceur secondaire de la figure 7, dans lequel l'implant secondaire est disposé de façon compacte avant sa pose,
- la figure 11 illustre le détail de la figure 10 avec l'implant secondaire libéré et déployé,
- la figure 12 illustre le lanceur principal conforme à l'invention avec l'implant principal dans un état déployé,
- la figure 13 est une illustration partielle d'un agrandissement de la figure 12,

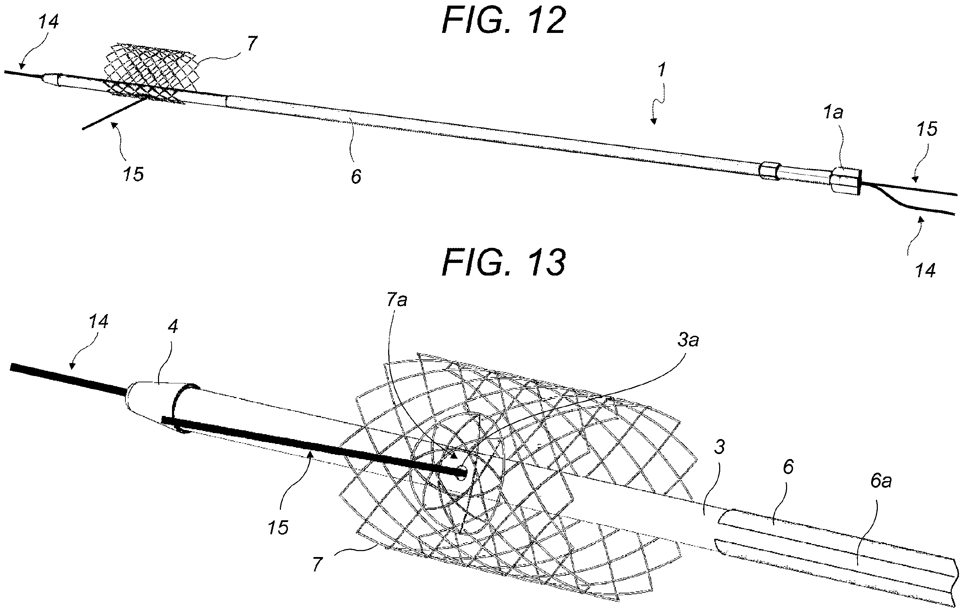

- les figures 13 à 25 illustrent schématiquement le procédé de pose et de connexion des implants au niveau d'une bifurcation de conduits par l'intermédiaire de l'ensemble conforme à l'invention,

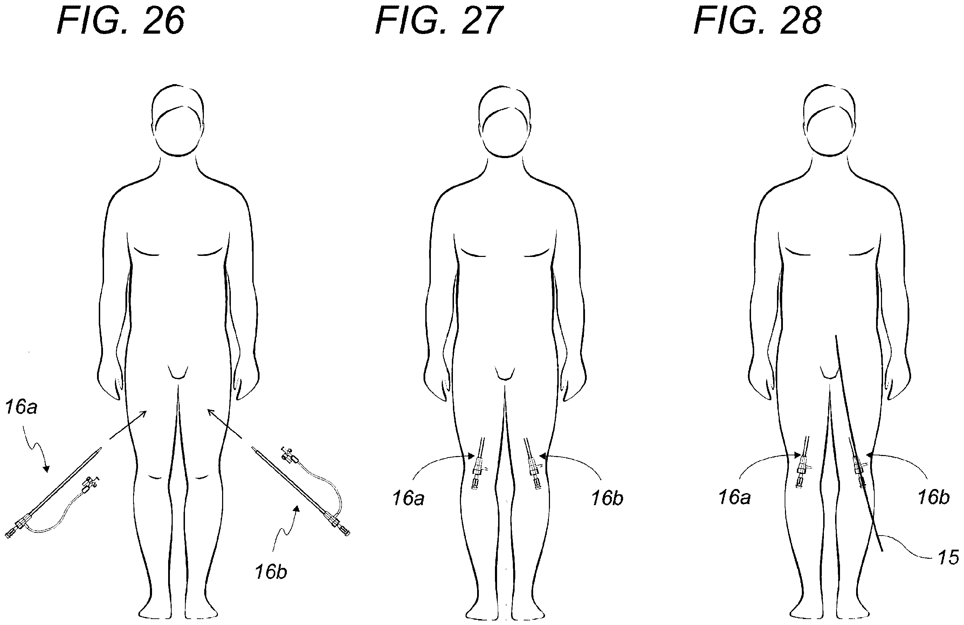

- les figures 26 à 31, illustrent, avec une localisation anatomique, certaines des étapes illustrées aux figures 13 à 25,

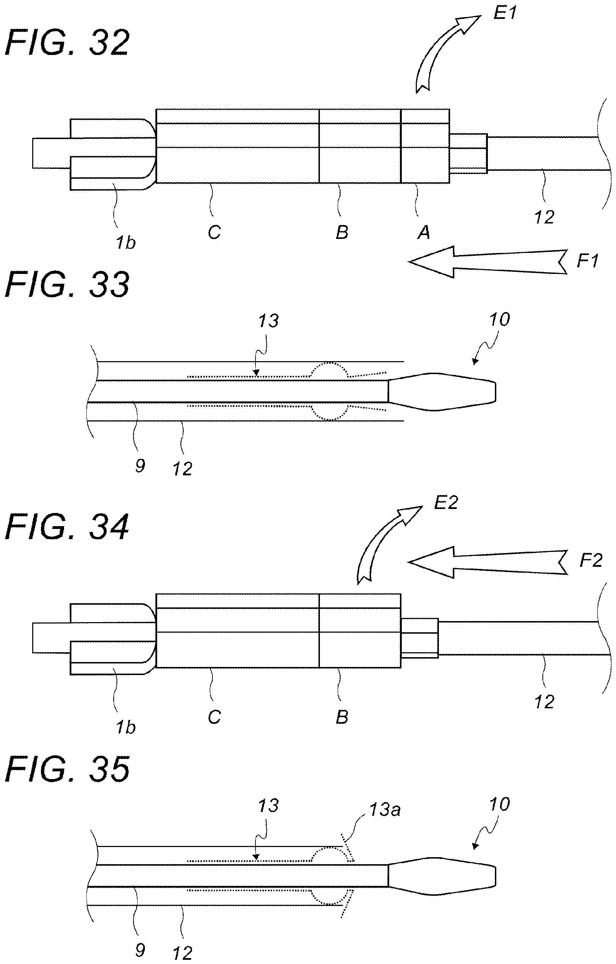

- la figure 32 illustre un exemple de réalisation de la configuration de l'extrémité proximale de manipulation d'un lanceur secondaire de l'ensemble conforme à l'invention, avant la pose d'un implant secondaire,
- la figure 33 illustre schématiquement et en coupe l'extrémité distale du lanceur secondaire dans la configuration de la figure 32,
- les figures 34 à 39 illustrent les étapes de libération de l'implant secondaire par l'intermédiaire de représentations respectives des extrémités proximales (figures 34, 36, 38) et distales (figures 35, 37, 39) du lanceur secondaire de l'ensemble conforme à l'invention, et

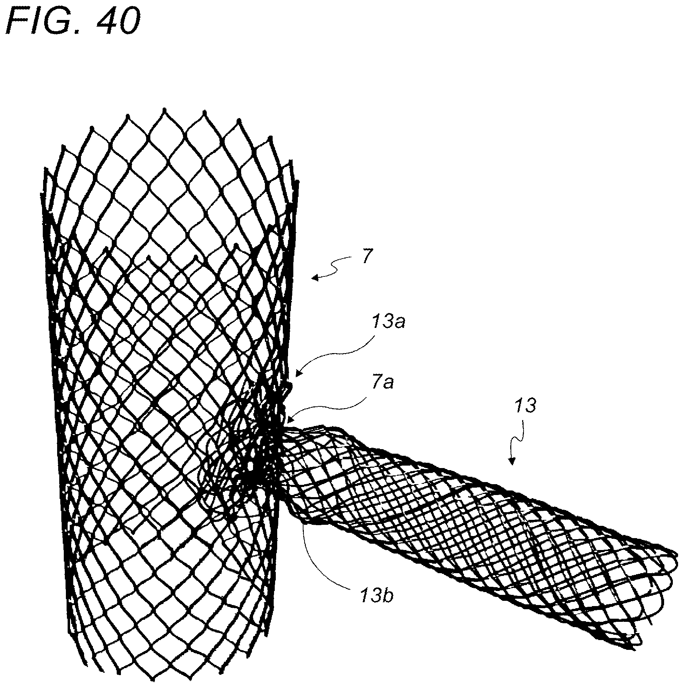

- la figure 40 représente un exemple de réalisation d'une connexion entre un implant principal et un implant secondaire, obtenue à l'aide d'un ensemble conforme à la mention.

### Exposé détaillé de l'invention

Les éléments structurellement et fonctionnellement identiques et présents sur différentes figures conservent la même référence numérique ou alphanumérique.

L'invention concerne un ensemble pour la mise en place et la connexion d'au moins deux d'implants dans des conduits de circulation principal et secondaire d'un fluide corporel, en communication fluidique via une bifurcation. Le conduit de circulation secondaire est aussi appelé conduit branche. La bifurcation peut présenter des conduits principal C1 et secondaire C2 s'étendant sensiblement orthogonalement ou avec une inclinaison déterminée.

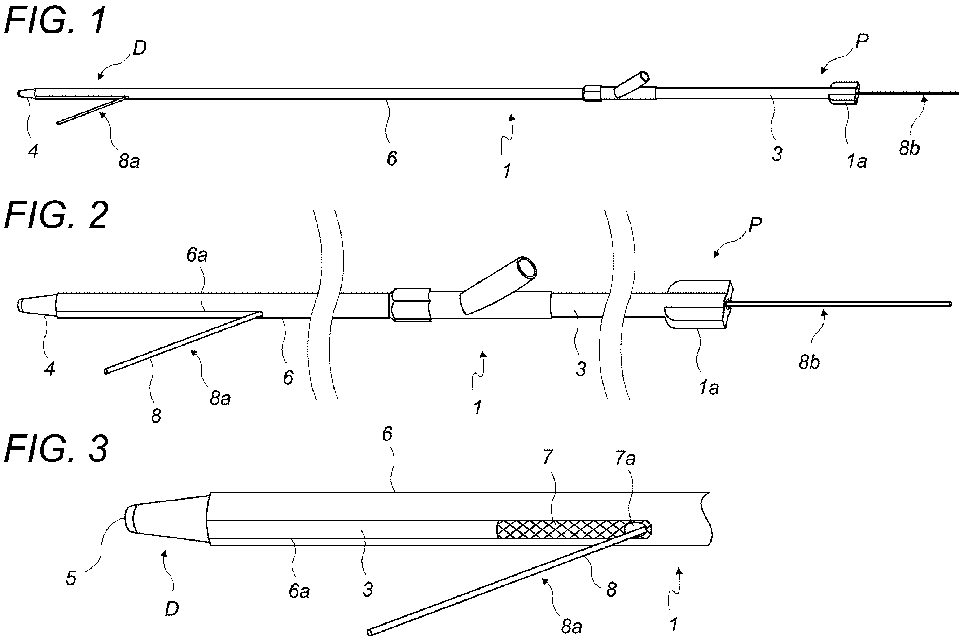

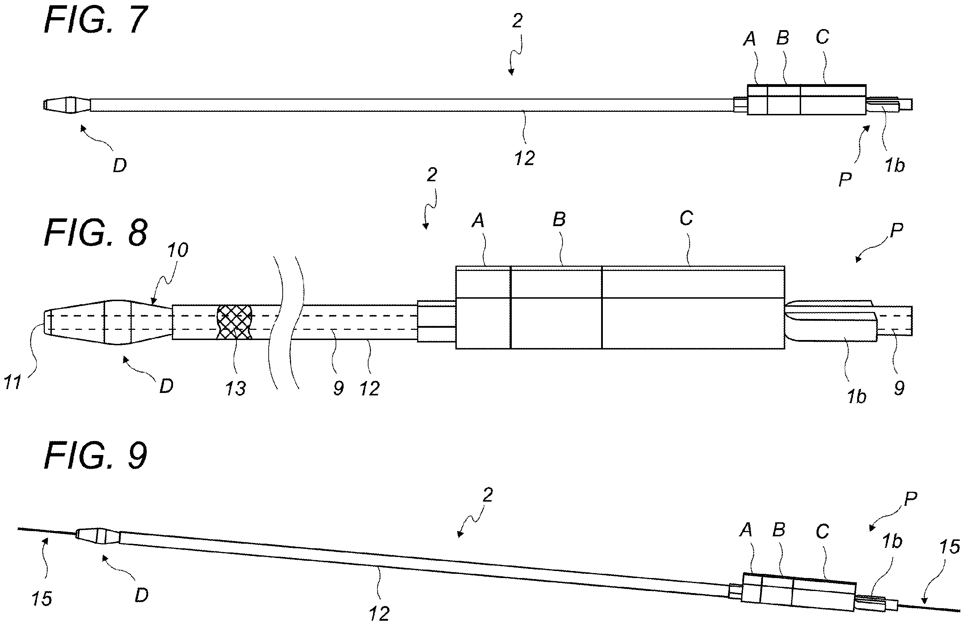

L'ensemble comporte un lanceur principal 1 et un lanceur secondaire 2, le conduit principal présentant avec le conduit secondaire une bifurcation. Les figures 1 à 6, 12 et 13 sont des illustrations d'exemples de réalisation du lanceur principal 1 conforme à l'invention, tandis que les figures 7 à 11 sont des illustrations d'un exemple de réalisation du lanceur secondaire 2 de l'ensemble conforme à l'invention.

Les lanceurs principal 1 et secondaire 2 présentent chacun une extrémité distale D et une extrémité proximale P de manipulation comportant respectivement un point fixe 1a et 1b.

Le lanceur principal 1 comporte une gaine intérieure 3 pourvue à son extrémité distale d'une pointe a-traumatique 4 avec un trou axial 5 communiquant avec un tube formé par ladite gaine intérieure 3. Le lanceur principal 1 comporte également une gaine extérieure principale 6. Au moins un implant principal 7 est disposé de façon non déployé entre les gaines extérieure 6 et intérieure 3.

La gaine extérieure 6 est pourvue d'une rainure ou fente longitudinale 6a sous forme d'ouverture, sur une partie de sa longueur et ouverte à son extrémité au voisinage de la pointe a-traumatique 4. On pourra se reporter également aux figures 3 et 13, montrant l'implant principal 7, respectivement non déployé et déployé après un retrait par coulissement de la gaine extérieure principale 6.

La gaine intérieure 3 présente avantageusement un trou latéral 3a, radial et débouchant en regard de ladite rainure ou fente longitudinale 6a. On pourra se reporter notamment à la figure 13.

L'implant principal 7, monté sur la gaine intérieure 3, présente au moins une fenêtre 7a alignée avec le trou latéral 3a précité et avec la rainure ou fente longitudinale 6a. On pourra se reporter par exemple à la figure 3.

Le lanceur principal 1 comporte également une gaine complémentaire 8 dont une première extrémité 8a traverse le trou latéral 3a, la fenêtre 7a de l'implant principal 7 et la rainure longitudinale 6a. Une seconde extrémité 8b de la gaine complémentaire 8 sort du lanceur principal 1 au voisinage de son extrémité proximale P.

Le lanceur secondaire 2 comporte une gaine secondaire intérieure 9, illustrée schématiquement à la figure 8 et pourvue à son extrémité distale D d'une pointe a-traumatique secondaire 10 avec un trou axial secondaire 11 communiquant avec un tube interne formé par la gaine secondaire intérieure 9. Le lanceur secondaire 2 comporte également une gaine secondaire extérieure 12. Au moins un implant secondaire 13 est disposé de façon non déployé entre les gaines secondaires extérieure 12 et intérieure 9.

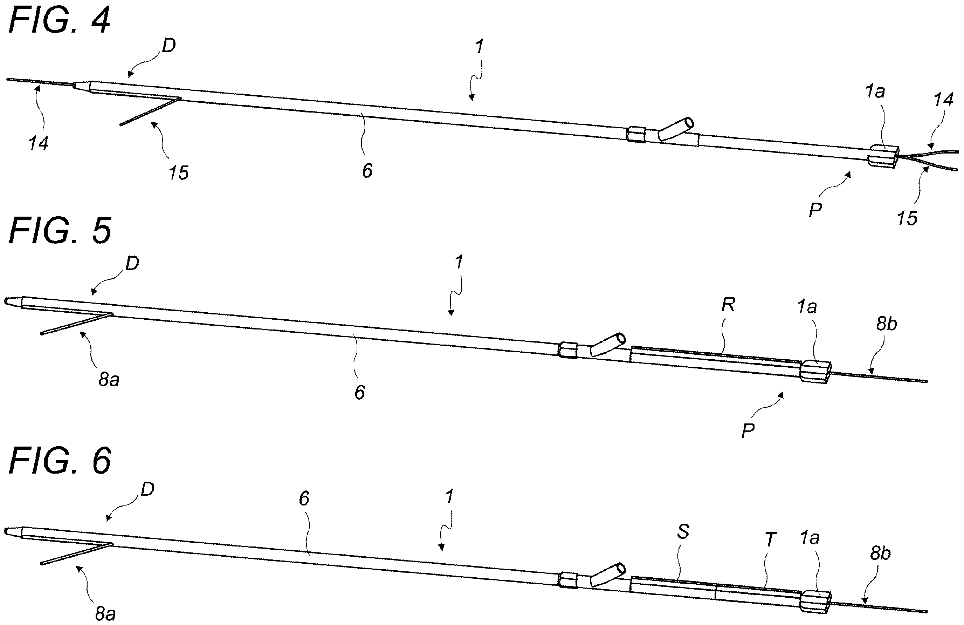

En se reportant également aux figures 4 et 13, il apparaît que l'ensemble conforme à l'invention comporte également un guide principal 14 pré-implantable dans conduit principal C1 du patient et destiné à s'engager dans le trou axial 5 à des fins de guidage du lanceur principal 1.

L'ensemble conforme à l'invention comporte également un guide secondaire 15 pré-implantable dans le conduit principal C1 et dans un conduit secondaire C2 en traversant la bifurcation formée par lesdits conduits C1, C2, pour s'engager dans la gaine complémentaire 8 à des fins de positionnement du lanceur principal 1. En outre, ce guide secondaire 15 permet de guider le lanceur secondaire 2 en vue du positionnement de l'implant secondaire 13. De tels guides 14, 15 sont connus entant que tel et ne seront donc pas décrits davantage.

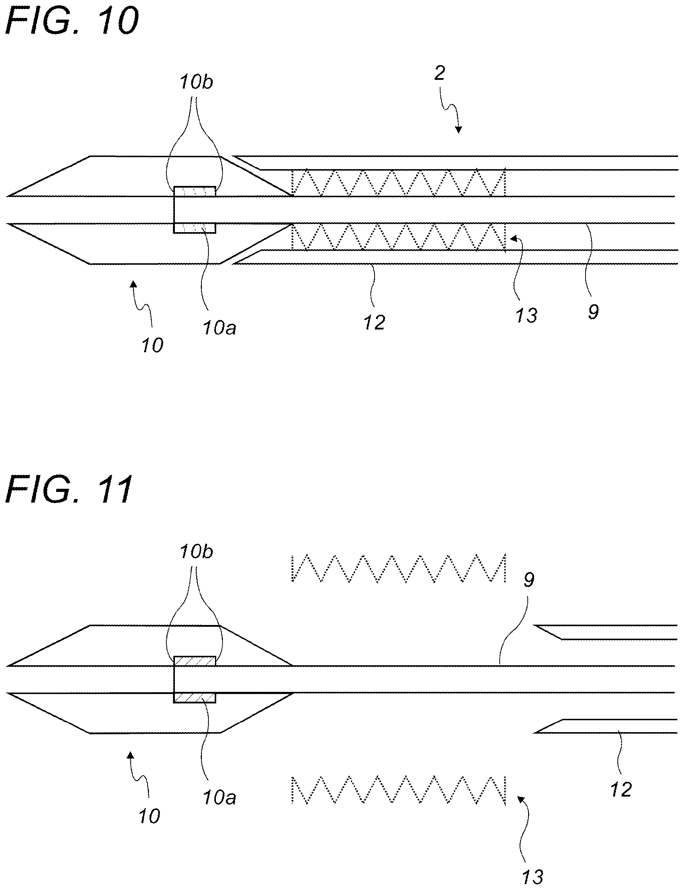

La figure 10 est une illustration schématique agrandie d'un détail du lanceur secondaire 2 des figures 7, 8 et 9, dans lequel l'implant secondaire 13, pouvant être appelé également implant branche, est disposé avant sa pose, de façon non déployé entre les gaines secondaires extérieure 12 et intérieure 9.

La figure 11 illustre le détail de la figure 10 avec l'implant secondaire 13, par exemple auto-expansible, libéré et déployé in situ dans le conduit secondaire C2, après le coulissement de la gaine extérieure 12 vers le point fixe 1b du lanceur secondaire 2.

Selon un exemple de réalisation avantageux, conforme à l'invention, la pointe a-traumatique secondaire 10 du lanceur secondaire 2 est une pointe à double cône. Une telle conformation réduit substantiellement les risques d'accrochage aux implants lors de la manipulation du lanceur secondaire 2 et notamment lors du retrait de celui-ci du conduit secondaire C2.

La pointe a-traumatique secondaire 10 est maintenue sur la gaine intérieure secondaire 9 grâce à une bague 10a solidaire de ladite gaine intérieure secondaire 9. La bague 10a est par exemple fixée sur la gaine intérieure secondaire 9 par collage, thermo-rétractation ou par tout autre moyen connu. La pointe a-traumatique secondaire 10 présente par ailleurs un logement interne délimitant des épaulements 10b entre lesquels vient se loger la bague 10a, empêchant tout coulissement sur la gaine intérieure secondaire 9 et par conséquent empêchant une séparation.

Selon un exemple de réalisation conforme à l'invention, illustré par exemple à la figure 5, la gaine extérieure principale 6 est montée coulissante sur la gaine intérieure 3 et bloquée en coulissement par la pointe a-traumatique 4 d'une part et par une butée amovible R d'autre part.

Selon un autre exemple de réalisation conforme à l'invention, illustré à la figure 6, la gaine extérieure principale 6 est montée coulissante sur la gaine intérieure 3 et bloquée en coulissement par la pointe a-traumatique 4 d'une part et par deux butée amovible successives S, T d'autre part.

Les butées R ou S et T sont bloquées en coulissement entre l'extrémité proximale de la gaine extérieure principale 6 et le point fixe la.

Selon un exemple de réalisation conforme à l'invention, la gaine extérieure secondaire 12 est montée coulissante sur la gaine intérieure secondaire 9 et bloquée en coulissement par la pointe a-traumatique secondaire 10 d'une part et par trois butées successives et amovibles A, B, C d'autre part.

Les butées A, B, C sont bloquées en coulissement entre l'extrémité proximale de la gaine extérieure secondaire 12 et le point fixe 1b.

Les butées successives A, B et C assurent, par leur séparation avec le lanceur secondaire 2, une libération progressive de l'implant secondaire 13 dans le conduit secondaire C2 et par conséquent sa connexion sur l'implant principal 7 logé dans le conduit principal C1.

Selon un autre exemple de réalisation conforme à l'invention, non représenté aux figures, la gaine extérieure secondaire 12 est montée coulissante sur la gaine intérieure secondaire 9 et bloquée en coulissement par la pointe a-traumatique secondaire 10 d'une part et par deux butées successives A, B et amovibles A, B d'autre part.

Selon un exemple de réalisation conforme à l'invention, l'implant secondaire 13 est conformé pour présenter des organes de connexion 13a, 13b coopérant avec l'implant principal 7. Les organes de connexion 13a et 13b réalisent automatiquement la connexion avec l'implant principal 7 lors de la libération et du déploiement dudit implant secondaire 13. On pourra se reporter également aux figures 32 à 39.

Selon un exemple de réalisation conforme à l'invention, la gaine complémentaire 8 est avantageusement extractible du lanceur principal 1. La gaine complémentaire 8 est ainsi retirée du lanceur principal 1 lorsque le guide secondaire 15 est mis en place dans les conduits C1, C2 et dans le lanceur principal 1 en traversant ladite gaine complémentaire 8. Cette dernière permet une introduction du guide secondaire 15, appelé aussi guide latéral dans le lanceur principal 1 et notamment d'assurer le passage facile dans la fenêtre 7a de l'implant principal 7 non déployé et le passage facile du trou latéral 3a de la gaine intérieure 3. Cette opération peut ainsi être effectuée rapidement avec une grande fiabilité.

Selon un exemple de réalisation conforme à l'invention, non représenté aux figures, le lanceur secondaire 2 présente une pointe a-traumatique secondaire 10 incurvée. Une telle forme permet de mieux orienter la direction d'approche de l'implant secondaire 13 vis-à-vis de la fenêtre 7a ménagée dans l'implant principal 7. Ceci peut s'avérer utile pour certaines formes de bifurcations et pour certains rayons de courbure rencontrés.

Selon un exemple de réalisation conforme à l'invention, le lanceur secondaire 2 présente avantageusement une portion d'extrémité réalisée avec un matériau flexible. Avantageusement, cette portion d'extrémité peut présenter une forme courbe.

L'ensemble conforme à l'invention permet de mettre en œuvre un nouveau procédé de pose d'implants 7, 13 particulièrement simple, précis et fiable. En effet le procédé de pause permet de s'assurer que les implants 7 et 13 sont bien positionnés et orientés à l'intérieur des conduits C1, C2. Ceci favorise une connexion rapide et fiable entre eux desdits implants 7, 13. Les figures schématiques 14 à 25, les figures 26 à 31 ainsi que les figures 32 à 39, illustrent un exemple de mise en œuvre d'un tel nouveau procédé.

Les étapes se rapportant à la préparation du patient ne sont pas décrites en détail dans la présente. Ces étapes préparatoires sont en effet largement connues. Il s'agit notamment de la pose d'introducteurs 16a et 16b sur le patient. On pourra se reporter aux figures 26 et 27.

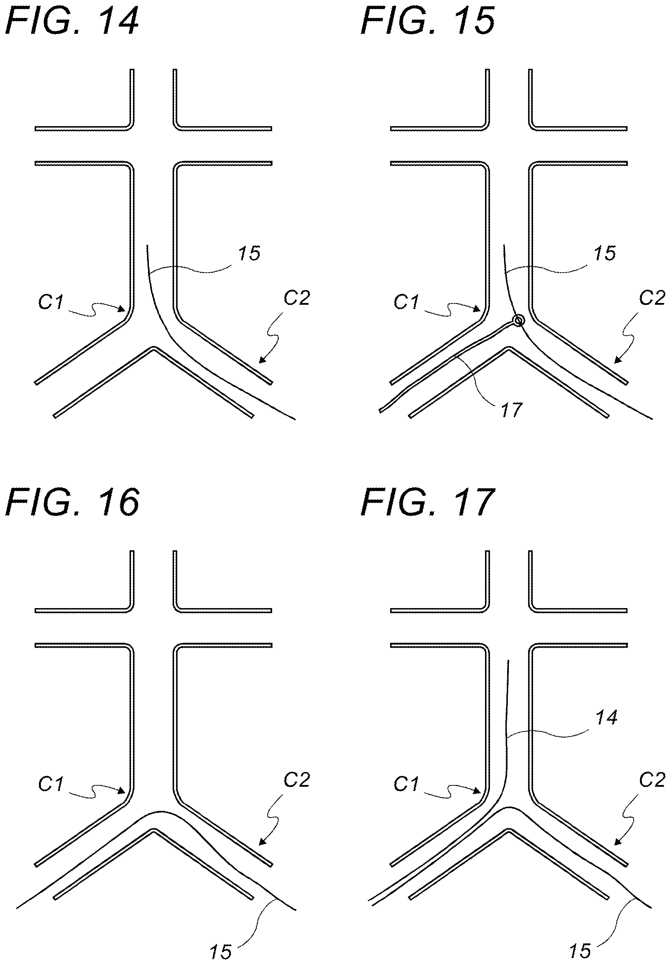

Ainsi, le procédé mis en œuvre à l'aide de l'ensemble conforme à l'invention consiste à engager le guide secondaire 15, pouvant aussi être appelé guide latéral, dans le conduit secondaire C2 en utilisant un introducteur 16b. On pourra se reporter aux figures 14 et 28.

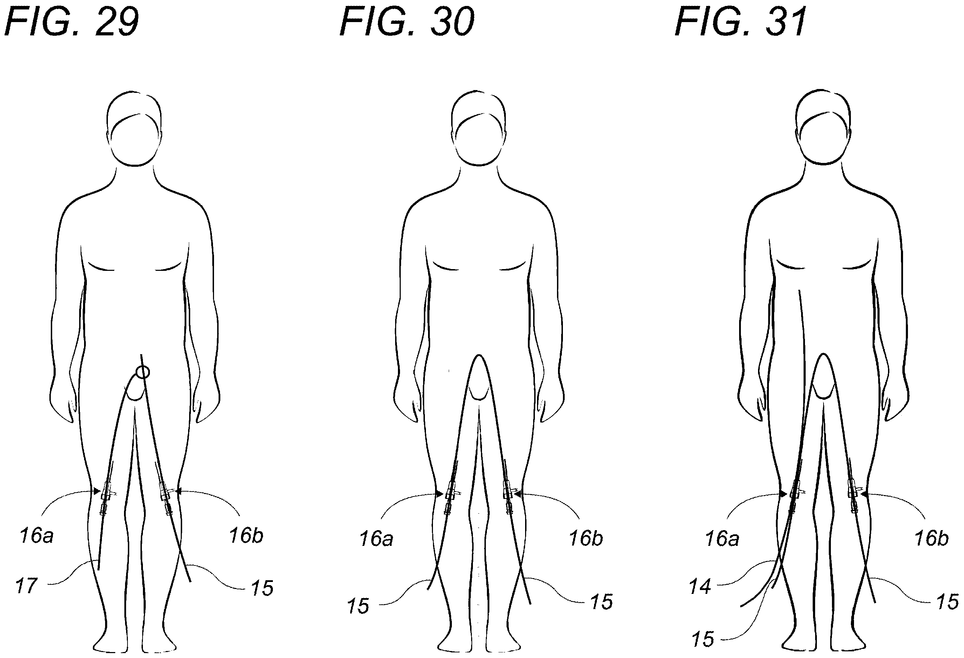

Ensuite, le procédé consiste à introduire dans le conduit principal C1, via un autre introducteur 16a, un organe de préhension 17, du genre lasso, pour récupérer le guide secondaire 15 au niveau de la bifurcation entre les conduits C1 et C2. On pourra se reporter aux figures 15 et 29.

L'organe de préhension 17 permet ainsi d'accrocher le guide secondaire 15 et de le faire passer dans le conduit principal C1 et de l'extraire de celui-ci par l'une de ses extrémités au niveau de l'autre introducteur 16a. On pourra se reporter aux figures 16 et 30.

Les figures 17 et 31 illustrent le positionnement du guide secondaire 15 lequel présente une première portion s'étendant dans le conduit principal C1 et sortant de ce dernier avec une de ces extrémités via l'autre introducteur 16a. Le guide secondaire 15 s'étend également dans le conduit secondaire C2 et sort de ce dernier avec l'autre de ces extrémités via l'introducteur 16b.

Les figures 17 et 31 illustrent également une étape complémentaire consistant à engager le guide principal 14, pouvant aussi être appelé guide axial, dans le conduit principal C1 en utilisant l'autre introducteur 16a.

L'extrémité du guide secondaire 15 sortant par une incision du côté conduit principal C1 et par l'autre introducteur 16a est ensuite engagée dans la première extrémité 8a de la gaine complémentaire 8 du lanceur principal 1.

Le guide principal 14 et plus précisément son extrémité sortant de l'autre introducteur 16a, est ensuite engagé dans le lanceur principal 1 par le trou axial 5.

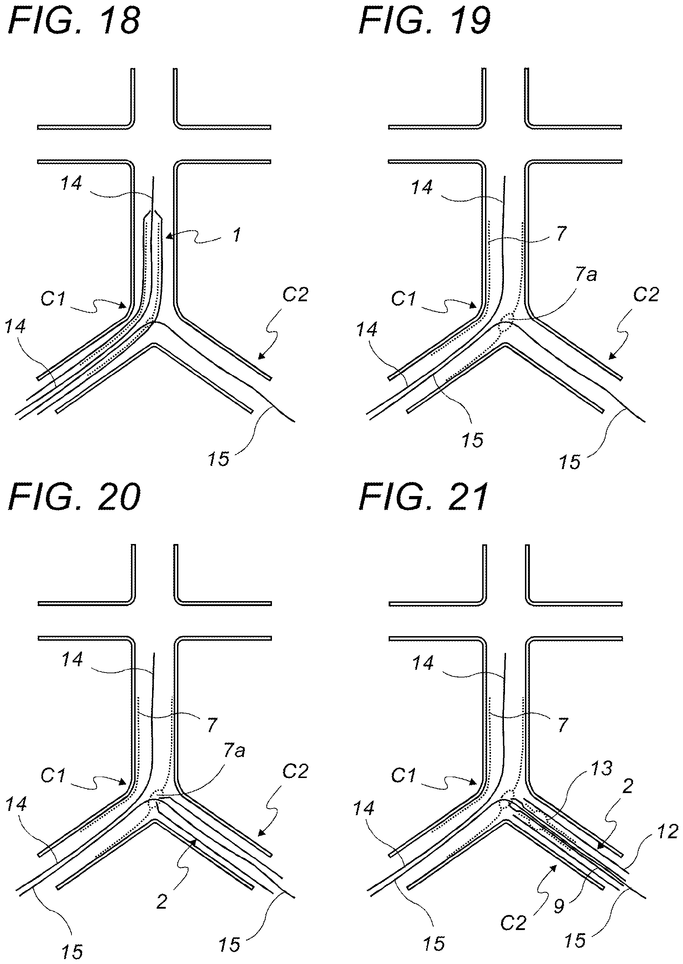

La gaine complémentaire 8 est ensuite retirée du lanceur principal 1. Ce dernier est ensuite introduit dans le conduit principal C1, via l'autre introducteur 16a, pour être positionné au niveau de la bifurcation de manière à ce que le trou latéral 3a et la fenêtre 7a de l'implant principal 7 se retrouvent sensiblement dans le prolongement du conduit secondaire C2. Cette position est facilement identifiable lors du guidage du lanceur principal 1 par le guide principal 14, grâce à la localisation radio-opaque du guide secondaire 15. Le guide principal 14 permet principalement de guider l'avancée du lanceur principal 1 dans le conduit principal C1. On pourra se reporter notamment à la figure 18.

La figure 19 illustre ensuite l'étape lors de laquelle la gaine extérieure 6 est coulissée en direction d'un point fixe la du lanceur principal 1 de manière à libérer l'implant principal 7. Ce dernier se déploie ainsi au niveau de la bifurcation. La libération de l'implant principal 7 est obtenue par exemple par le retrait d'une butée R du lanceur principal 1 de la figure 5. Selon un autre exemple de mise en œuvre, la libération de l'implant principal 7 est obtenue par le retrait successif des deux butées S, T juxtaposées du lanceur principal 1 illustré à la figure 6.

Une fois l'implant principal 7 entièrement déployé, comme illustré à la figure 19 le lanceur principal 1 est retiré du conduit principal C1.

Ensuite lors d'une étape supplémentaire, le procédé consiste à introduire via l'introducteur 16b, le lanceur secondaire 2, guidé par le guide secondaire 15, dans le conduit secondaire C2 et ce jusqu'au niveau de la fenêtre 7a ménagée dans l'implant principal 7. Cette fenêtre 7a se situe bien entendue au niveau de la bifurcation et est traversée par le guide secondaire 15. On pourra se reporter à la figure 20.

Les figures 21 à 25 et 32 à 39 illustrent ensuite la libération de l'implant secondaire 13 ainsi que sa connexion sur l'implant principal 7 lors d'opérations successives.

Ainsi les figures 21 et 33 illustrent le positionnement du lanceur secondaire 2 avec la gaine extérieure secondaire 12, la gaine intérieure secondaire 9, l'implant secondaire 13 et le guide secondaire 15 avant déploiement et libération dudit implant secondaire 13.

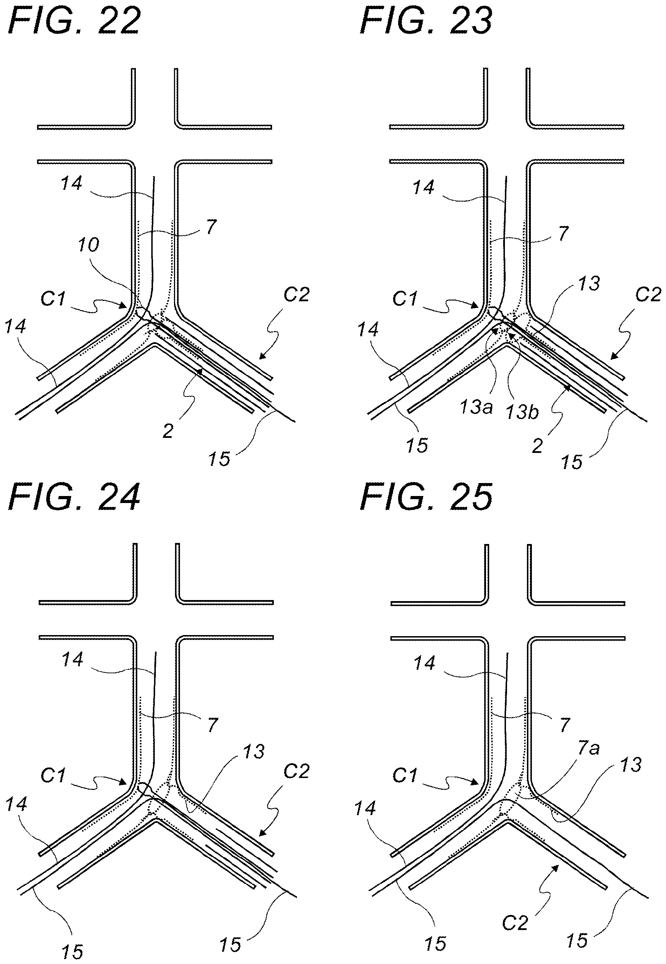

Le procédé consiste ensuite à retirer la première butée A du lanceur secondaire 2 (flèche E1) et à faire coulisser (flèche F1) la gaine extérieure secondaire 12 contre la seconde butée B dudit lanceur latéral secondaire 2. Ceci permet de libérer une première extrémité de l'implant secondaire 13 et notamment les organes de connexion 13a au-delà de la fenêtre 7a de l'implant principal 7. Cette étape est également illustrée à l'aide des figures 22 et 35. Les organes de connexion 13a sont par exemple constitués par une portion d'extrémité repliable 13a de l'implant secondaire 13.

Ensuite, selon une étape supplémentaire, le procédé consiste à retirer la seconde butée B du lanceur secondaire 2 (flèche E2) et faire coulisser (flèche F2) la gaine extérieure secondaire 12 jusqu'à la troisième butée C de manière à libérer également un bourrelet 13b de positionnement de l'implant secondaire 13.

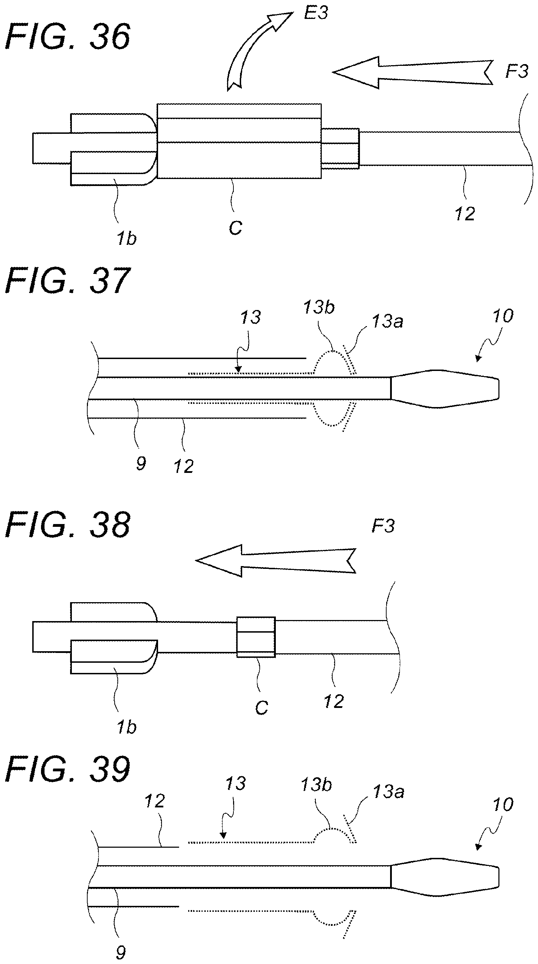

Ce bourrelet 13b est situé cependant avant la fenêtre 7a de l'implant principal 7. Le bourrelet 13b et la portion d'extrémité repliable 13a, permettent ainsi de bloquer l'implant secondaire 13 sur l'implant principal 7 au niveau de la fenêtre 7a. On évite ainsi une obstruction partielle du conduit principal C1 par une extrémité dudit implant secondaire 13. Ce dernier ne peut pas en effet venir en saillie dans l'implant principal 7. Cette étape est également illustrée par exemple à l'aide des figures 23 et 37.

Le procédé consiste ensuite à retirer la troisième butée C (flèche E3) et à faire coulisser (flèche F3) la gaine extérieure secondaire 12 vers le point fixe 1b du lanceur secondaire 2. Ceci correspond à une libération et à un déploiement complet de l'implant secondaire 13 ainsi qu'à une connexion de ce dernier sur l'implant principal 7 au niveau de la fenêtre 7a. On pourra se reporter par exemple aux figures 24, 36, 38 et 39.

Une étape complémentaire consiste ensuite à retirer le guide principal 14 et le guide secondaire 15 des conduits respectifs C1 et C2.

On obtient ainsi un implant secondaire 13 et un implant principal 7 connectés au niveau d'une bifurcation entre un conduit principal C1 et un conduit secondaire C2, comme cela est par exemple représenté aux figures 25 et 40.

De manière évidente, l'invention ne se limite pas au mode de réalisation préférentiel décrit précédemment et illustré par les différentes figures, l'homme du métier pouvant y apporter de nombreuses modifications et imaginer d'autres exemples de réalisation et/ou de mise en œuvre, sans sortir ni de la portée, ni du cadre de l'invention définis par les revendications.

## Revendications

1. Lanceur principal (1) comportant au moins un implant principal (7) pour la mise en place dudit au moins un implant principal (7) connectable à un autre implant au niveau d'une bifurcation entre un conduit principal (C1) et un conduit secondaire (C2) de circulation principal d'un fluide corporel, ledit lanceur principal (1) présentant une extrémité distale D et une extrémité proximale P de manipulation comportant un point fixe (1a),
**caractérisé en ce que**:
- ledit lanceur (1) comporte une gaine intérieure principale (3) pourvue à son extrémité distale D d'une pointe a-traumatique (4) avec un trou axial (5) communiquant avec un tube longitudinal formé par ladite gaine intérieure (3),
- ledit lanceur (1) comportant une gaine extérieure principale (6),
- ledit au moins un implant principal (7) étant disposé entre les gaines extérieure (6) et intérieure (3) principales,
- la gaine extérieure principale (6) étant pourvue d'une rainure ou fente longitudinale (6a) s'étendant sur une partie de sa longueur,
- la gaine intérieure principale (3) présentant un trou latéral (3a) débouchant en regard de ladite rainure ou fente longitudinale (6a),
- l'implant principal (7) présentant au moins une fenêtre (7a) alignée avec le trou latéral (3a),
- et ledit lanceur (1) comportant au moins une gaine complémentaire (8) dont une première extrémité (8a) traverse le trou latéral (3a), la fenêtre (7a) de l'implant principal (7) et la rainure ou fente longitudinale (6a) et dont une seconde extrémité (8b) sort au voisinage de l'extrémité proximale P, ladite gaine complémentaire (8) étant destinée à être traversée par un guide.

2. Lanceur (1) selon la revendication 1, **caractérisé en ce que** la gaine extérieure principale (6) est montée coulissante sur la gaine intérieure principale (3) et bloquée en coulissement par la pointe a-traumatique (4) d'une part et par au moins une butée amovible R, S, T) d'autre part, ladite rainure ou fente longitudinale (6a) étant ouverte à son extrémité distale D au voisinage de la pointe a-traumatique (4).

3. Lanceur (1) selon la revendication 1, **caractérisé en ce que** la gaine extérieure principale (6) est constituée d'un matériau pelable.

4. Lanceur (1) selon l'une quelconque des revendications 1 à 3, **caractérisé en ce que** la gaine complémentaire (8) est extractible du lanceur (1) et permet l'introduction d'un guide secondaire (15) dans ledit lanceur (1) et permet d'assurer le passage dans la fenêtre (7a) de l'implant principal (7) et du trou latéral (3a) de la gaine intérieure principale (3).

5. Ensemble pour la mise en place et la connexion d'au moins deux implants (7, 13) dans des conduits de circulation principal (C1) et secondaire (C2) d'un fluide corporel, le conduit principal (C1) présentant avec le conduit secondaire (C2) une bifurcation, **caractérisé en ce qu'**il comprend :
- un lanceur (1) principal conforme à l'une quelconque des revendications 1 à 4,
- un lanceur secondaire (2), indépendant du lanceur (1) principal, présentant une extrémité distale D et une extrémité proximale P de manipulation comportant un point fixe (1b),
- ledit lanceur secondaire (2) comportant une gaine secondaire intérieure (9) pourvue à son extrémité distale D, d'une pointe a-traumatique secondaire (10) avec un trou axial secondaire (11) communiquant avec un tube longitudinal formé par ladite gaine secondaire intérieure (9), une gaine secondaire extérieure (12), au moins un implant secondaire (13) disposé entre les gaines secondaires extérieure (12) et intérieure (9).

6. Ensemble selon la revendication 5, **caractérisé en ce qu'**il comprend un guide principal (14) pré-implantable dans le conduit principal (C1) et destiné à s'engager dans le trou axial (5) à des fins de guidage du lanceur principal (1).

7. Ensemble selon la revendication 5 ou 6, **caractérisé en ce qu'**il comprend un guide secondaire (15) pré-implantable dans le conduit principal (C1) et dans le conduit secondaire (C2) en traversant la bifurcation, pour s'engager dans la gaine complémentaire (8) à des fins de positionnement du lanceur principal (1) et/ou à des fins de guidage du lanceur secondaire (2).

8. Ensemble selon l'une quelconque des revendications 5 à 7, **caractérisé en ce que** la pointe a-traumatique secondaire (10) du lanceur secondaire (2) est une pointe à double cône.

9. Ensemble selon l'une quelconque des revendications 5 à 8, **caractérisé en ce que** la gaine extérieure secondaire (12) est montée coulissante sur la gaine intérieure secondaire (9) et bloquée en coulissement par la pointe a-traumatique secondaire (10) d'une part et par au moins deux butées successives et amovibles (A, B) d'autre part, lesdites butées (A, B) assurant la libération progressive de l'implant secondaire (13) ainsi que sa connexion sur l'implant principal (7).

10. Ensemble selon la revendication 9, **caractérisé en ce que** la gaine extérieure secondaire (12) est montée coulissante sur la gaine intérieure secondaire (9) et bloquée en coulissement par la pointe a-traumatique secondaire (10) d'une part et par trois butées successives et amovibles (A, B, C) d'autre part.

11. Ensemble selon l'une quelconque des revendications 5 à 10, **caractérisée en ce que** l'implant secondaire (13) est conformé pour présenter des organes de connexion (13) à l'implant principal (7), lesquels forment la connexion avec l'implant principal (7) automatiquement lors de la libération et du déploiement dudit implant secondaire (13).

12. Ensemble selon l'une quelconque des revendications 5 à 11, **caractérisé en ce que** la pointe a-traumatique secondaire (10) présente une extrémité distale D incurvée.

13. Ensemble selon l'une quelconque des revendications 5 à 12, **caractérisé en ce que** la pointe a-traumatique secondaire (10) est réalisée avec un matériau flexible.

14. Ensemble selon l'une quelconque des revendications 5 à 13, **caractérisé en ce que** les implants (7, 13) sont reliés à leur gaine intérieure correspondante (3, 9) par l'intermédiaire d'un organe de retenue permettant de retirer et/ou de repositionner dans le lanceur (1, 2) lesdits implants en cas de nécessité lors de l'opération de pose.

## Patentansprüche

1. Hauptinjektor (1), der mindestens ein Hauptimplantat (7) enthält, zum Einsetzen dieses mindestens einen Hauptimplantats (7), das an ein anderes Implantat anschließbar ist, an einer Verzweigung zwischen einem Hauptkreislauf (C1) und einem Sekundärkreislauf (C2) eines Hauptkreislaufs einer Körperflüssigkeit, der erwähnte Hauptinjektor (1) weist dabei ein distales Endstück D und ein proximales Endstück P zur Bedienung auf, mit einem festen Punkt (1a), **dadurch gekennzeichnet, dass**:
- der erwähnte Injektor (1) eine Hauptinnenhülse (3) enthält, versehen an seinem distalen Endstück D mit einer atraumatischen Spitze (4) mit einem axialen Loch (5), das mit einem länglichen Rohr kommuniziert, das von dieser Innenhülse (3) gebildet wird,
- der erwähnte Injektor (1) eine Hauptaußenhülse (6) enthält,
- das erwähnte mindestens eine Hauptimplantat (7) dabei zwischen der Außen- (6) und Innenhülse (3) angeordnet ist,
- die Außenhülse (6) mit einer Rille oder einem Längsschlitz (6a) versehen ist, die über einen Teil der Länge verläuft,
- die Hauptinnenhülse (3) ein seitliches Loch (3a) aufweist, das gegenüber dieser Rille oder diesem Längsschlitz (6a) mündet.
- das Hauptimplantat (7) mindestens ein Fenster (7a) aufweist, das an dem seitlichen Loch (3 a) ausgerichtet ist,
- und der erwähnte Injektor (1) mindestens eine komplementäre Hülse (8) enthält, deren erstes Endstück (8a) durch das seitliche Loch (3a), das Fenster (7a) des Hauptimplantats (7) und die Rille oder den Längsschlitz (6a) hindurchführt und deren zweites Endstück (8b) in der Nähe des proximalen Endstücks P herauskommt, diese komplementäre Hülse (8) ist für die Hindurchführung einer Führung bestimmt.

2. Injektor (1) nach Anspruch 1, **dadurch gekennzeichnet, dass** die Hauptaußenhülse (6) gleitend auf der Hauptinnenhülse (3) montiert ist, das Gleiten wird durch die atraumatische Spitze (4) einerseits und von mindestens einem beweglichen Ansatz (R, S, T) andererseits blockiert, diese Rille oder Längsschlitz (6a) ist an ihrem distalen Endstück D in der Nähe der atraumatischen Spitze (4) offen.

3. Injektor (1) nach Anspruch 1, **dadurch gekennzeichnet, dass** die Außenhülse (6) aus einem abziehbaren Material besteht.

4. Injektor (1) nach irgendeinem der vorangehenden Ansprüche 1 bis 3, **dadurch gekennzeichnet, dass** die komplementäre Hülse (8) aus dem Injektor (1) herausziehbar ist und das Einführen einer zweiten Führung (15) in den erwähnten Injektor (1) erlaubt und es möglich macht, die Passage im Fenster (7a) des Hauptimplantats (7) und des seitlichen Lochs (3a) der Hauptinnenhülse (3) zu gewährleisten.

5. Bauteil zum Einsetzen und Verbindung von mindestens zwei Implantaten (7, 13) in den Haupt- (C1) und Sekundärkreisläufen (C2) einer Körperflüssigkeit, wobei der Hauptkreislauf (C1) mit dem Sekundärkreislauf (C2) eine Verzweigung bildet, **dadurch gekennzeichnet, dass** es enthält::
- einen Hauptinjektor (1) gemäß einem beliebigen der Ansprüche 1 bis 4,
- einen Sekundärinjektor (2), unabhängig vom Hauptinjektor (1), mit einem distalen Endstück D und einem proximalen Endstück P zur Bedienung, mit einem Festpunkt (1b),
- der erwähnte Sekundärinjektor (2) enthält dabei eine sekundäre Innenhülse (9), versehen an ihrem distalen Endstück D, mit einer sekundären atraumatischen Spitze (10) mit einem axialen, sekundären Loch (11), das mit einem länglichen Rohr kommuniziert, das von dieser sekundären Innenhülse (3) gebildet wird, eine sekundäre Außenhülse (12), mindestens ein sekundäres Implantat (13), angeordnet zwischen der sekundären Außen- (12) und Innenhülse (9).

6. Bauteil nach Anspruch 5, **dadurch gekennzeichnet, dass** sie eine Hauptführung (14) enthält, präimplantierbar in die Hauptführung (C1) und bestimmt zum Einrasten in dem axialen Loch (5), zum Zwecke der Führung des Hauptinjektors (1).

7. Bauteil nach Anspruch 5 oder 6, **dadurch gekennzeichnet, dass** sie eine sekundäre Führung (15) enthält, präimplantierbar in die Hauptführung (C1) und in die sekundäre Führung (C2) wobei sie durch die Verzweigung hindurchführt, um in der komplementären Hülse (8) zum Zwecke der Positionierung des Hauptinjektors (1) einzurasten und/ oder zum Zwecke der Führung des Sekundärinjektors (2).

8. Bauteil nach irgendeinem der vorangehenden Ansprüche 5 bis 7, **dadurch gekennzeichnet, dass** die sekundäre atraumatische Spitze (10) des Sekundärinjektors (2) eine Spitze mit Doppelkonus ist.

9. Bauteil nach irgendeinem der vorangehenden Ansprüche 5 bis 8, **dadurch gekennzeichnet, dass** die sekundäre Außenhülse (12) gleitend auf der sekundären Innenhülse (9) montiert ist, das Gleiten wird durch die sekundäre atraumatische Spitze (10) einerseits und von mindestens zwei aufeinanderfolgenden, beweglichen Anschlägen (A, B) andererseits blockiert, diese Anschläge (A, B) stellen die progressive Freisetzung des sekundären Implantats (13) sicher, sowie ihre Verbindung mit dem Hauptimplantat (7).

10. Bauteil nach Anspruch 9, **dadurch gekennzeichnet, dass** die sekundäre Außenhülse (12) gleitend auf der sekundären Innenhülse (9) montiert ist, das Gleiten wird durch die sekundäre atraumatische Spitze (10) einerseits und von mindestens drei aufeinanderfolgenden, beweglichen Anschlägen (A, B, C) andererseits blockiert.

11. Bauteil nach irgendeinem der vorangehenden Ansprüche 5 bis 10, **dadurch gekennzeichnet, dass** das sekundäre Implantat (13) so geformt ist, dass es Verbindungsorgane (13) mit dem Hauptimplantat (7) aufweist, die automatisch, bei der Freigabe und Entfaltung des erwähnten sekundären Implantats (13), die Verbindung mit dem Hauptimplantat (7) herstellen.

12. Bauteil nach irgendeinem der vorangehenden Ansprüche 5 bis 11, **dadurch gekennzeichnet, dass** die sekundäre atraumatische Spitze (10) ein gekrümmtes distales Endstück D aufweist.

13. Bauteil nach irgendeinem der vorangehenden Ansprüche 5 bis 12, **dadurch gekennzeichnet, dass** die sekundäre atraumatische Spitze (10) aus einem flexiblen Material besteht.

14. Bauteil nach irgendeinem der vorangehenden Ansprüche 5 bis 13, **dadurch gekennzeichnet, dass** die Implantate (7, 13) mit ihrer jeweiligen Innenhülse (3, 9) über eine Rückhaltevorrichtung verbunden sind, mit der es möglich ist, diese Implantate aus dem Injektor (1, 2) zu entfernen und/ oder sie während des Einsetzens neu zu positionieren.

## Claims

1. A main driver (1) having at least one main implant (7) for the insertion of said at least one main implant (7) connectable to another implant, at a bifurcation between a main conduit (C1) and a secondary conduit (C2) of circulation for a bodily fluid, said main driver (1) having a distal end D and a proximal handling end P having a fixed point (1a),
**characterized in that**:
- said driver (1) has a main internal sheath (3) provided at its distal end D with an atraumatic tip (4) with an axial hole (5) communicating with a longitudinal tube formed by said internal sheath (3),
- said driver (1) having a main external sheath (6),
- said at least one main implant (7) being disposed between the main external (6) and internal (3) sheaths,
- the main external sheath (6) being provided with a longitudinal groove or slit (6a) extending along a part of its length,
- the main internal sheath (3) having a lateral hole (3a) emerging facing said longitudinal groove or slit (6a),
- the main implant (7) having at least one window (7a) aligned with the lateral hole (3 a),
- and said driver (1) having at least one complementary sheath (8), a first end (8a) of which passes through the lateral hole (3a), the window (7a) of the main implant (7) and the longitudinal groove or slit (6a), and a second end (8b) of which comes out near the proximal end P, said complementary sheath (8) being intended to be passed through by a guide.

2. The driver (1) according to claim 1, **characterized in that** the main external sheath (6) is mounted sliding on the main internal sheath (3) and blocked in sliding by the atraumatic tip (4) on the one hand and by at least one removable stop (R, S, T) on the other hand, said longitudinal groove or slit (6a) being open at its distal end D near the atraumatic tip (4).

3. The driver (1) according to claim 1, **characterized in that** the main external sheath (6) is made of a peelable material.

4. The driver (1) according to any one of claims 1 to 3, **characterized in that** the complementary sheath (8) is extractable from the driver (1) and allows the insertion of a secondary guide (15) into said driver (1) and makes it possible to ensure the passage in the window (7a) of the main implant (7) and the passage of the lateral hole (3a) of the main internal sheath (3).

5. An assembly for the insertion and the connection of at least two implants (7, 13) in a main conduit (C1) and secondary conduit (C2) of circulation for a bodily fluid, the main conduit (C1) having a bifurcation with the secondary conduit (C2), **characterized in that** it comprises:
- a main driver (1) according to any one of claims 1 to 4,
- a secondary driver (2), independent of the main driver (1), having a distal end D and a proximal handling end P having a fixed point (1b),
- said secondary driver (2) having an internal secondary sheath (9) provided at its distal end D with a secondary atraumatic tip (10) with a secondary axial hole (11) communicating with a longitudinal tube formed by said internal secondary sheath (9), an external secondary sheath (12), at least one secondary implant (13) disposed between the external (12) and internal (9) secondary sheaths.

6. The assembly according to claim 5, **characterized in that** it comprises a main guide (14) pre-implantable in the main conduit (C1) and intended to engage in the axial hole (5) for guiding purposes of the main driver (1).

7. The assembly according to claim 5 or 6, **characterized in that** it comprises a secondary guide (15) preimplantable in the main conduit (C1) and in the secondary conduit (C2) while passing through the bifurcation, in order to engage in the complementary sheath (8) for positioning purposes of the main driver (1) and/or for guiding purposes of the secondary driver (2).

8. The assembly according to any one of claims 5 to 7, **characterized in that** the secondary atraumatic tip (10) of the secondary driver (2) is a double-cone tip.

9. The assembly according to any one of claims 5 to 8, **characterized in that** the secondary external sheath (12) is mounted sliding on the secondary internal sheath (9) and blocked in sliding by the secondary atraumatic tip (10) on the one hand and by at least two successive and removable stops (A, B) on the other hand, said stops (A, B) ensuring the gradual release of the secondary implant (13) as well as its connection on the main implant (7).

10. The assembly according to claim 9, **characterized in that** the secondary external sheath (12) is mounted sliding on the secondary internal sheath (9) and blocked in sliding by the secondary atraumatic tip (10) on the one hand and by three successive and removable stops (A, B, C) on the other hand.

11. The assembly according to any one of claims 5 to 10, **characterized in that** the secondary implant (13) is configured to have connection members (13) to the main implant (7), which automatically form the connection with the main implant (7) during the release and deployment of said secondary implant (13).

12. The assembly according to any one of claims 5 to 11, **characterized in that** the secondary atraumatic tip (10) has a curved distal end D.

13. The assembly according to any one of claims 5 to 12, **characterized in that** the secondary atraumatic tip (10) is made from a flexible material.

14. The assembly according to any one of claims 5 to 13, **characterized in that** the implants (7, 13) are connected to the corresponding internal sheath (3, 9) by means of a retaining member making it possible to remove and/or reposition said implants in the driver (1, 2) if needed during the insertion operation.
